# EUROPEAN PATENT APPLICATION

(11) **EP 3 203 815 A1**
(43) Date of publication of application: **09.08.2017**
(21) Application number: 16154282.4
(22) Date of filing: 04.02.2016
(51) Int. Cl.: H05H 7/00, A61N 5/00, G21K 1/00, H05H 7/12

(54) **ROTATING ENERGY DEGRADER**

(71) Applicant: Ion Beam Applications, 1348 Louvain-la-Neuve (BE)
(72) Inventor: DEBATTY, Alexandre, 1348 Louvain-la-Neuve (BE); GERARD, Nicolas, 1348 Louvain-la-Neuve (BE)
(74) Representative: De Groote, Christophe

(57) **Abstract**

An energy degrader (1) for attenuating the energy of a charged particle beam (2) and comprising : a first energy attenuation member (10) presenting a beam entry face (11) having the shape of a part of a first helical surface, a second energy attenuation member (20) presenting a beam exit face (22) having the shape of a part of a second helical surface, said beam exit face being positioned downstream of said beam entry face with respect to the beam direction, and a drive assembly (M1, M2) for rotating the first and/or the second energy attenuation members about respectively a first and/or a second rotation axis (A1, A2) while crossed by the particle beam. The first and second helical surfaces are continuous surfaces and have the same handedness, thereby allowing to build a more compact degrader with a smaller moment of inertia. More accurate and faster variation of the energy of the beam can hence be achieved.

## Description

### Field of the invention

The invention relates to the field of charged particle accelerators, such as proton or carbon ion accelerators for example, and more particularly to a rotating energy degrader for attenuating the energy of a charged particle beam extracted from such a particle accelerator.

The invention also relates to a particle therapy system comprising a particle accelerator and a rotating energy degrader for attenuating the energy of a charged particle beam extracted from the particle accelerator.

### Description of prior art

Certain applications involving the use of beams of charged particles require the energy of these particles to be varied. This is for example the case in particle therapy applications, where the energy of the charged particles determines the depth of penetration of these particles into a body to be treated by such therapy. Fixed-energy particle accelerators, such as cyclotrons for instance, are not themselves adapted to vary the energy of the particle beam which they produce, and therefore require an additional device to vary this energy. Variable-energy particle accelerators, such as synchrotrons for instance, are themselves adapted to vary the energy of the particle beam which they produce, but it may nevertheless be desirable to further vary the energy after the particles have been extracted from a synchrotron.

Devices for varying the energy of a particle beam extracted from a particle accelerator are generally called energy degraders. An energy degrader comprises therefore one or more blocks of matter which are placed across the path of the particle beam after its extraction from the particle accelerator and before the particle beam reaches a target. According to a well-known principle, a charged particle passing through the thickness of such a block of matter undergoes a decrease in its energy by an amount which is, for particles of a given type, a function of the intrinsic characteristics of the material passed through and of said thickness.

A known rotating energy degrader is for example disclosed in international patent publication WO-0038486. It comprises a single block of energy degrading material which has the shape of a double helicoidal staircase with discrete flat steps and which is placed across the path of the particle beam. The particle beam enters the degrader perpendicularly to a step at one side of the double staircase and exits the degrader perpendicularly to a step at the opposite side of the double staircase, which attenuates the energy of the beam according to the thickness of the degrader at said step. After having rotated the staircase by a given angle around its axis, the particle beam will enter the degrader perpendicularly to another step and exit the degrader at the opposite side, which will attenuate the energy of the beam by a different amount according to the thickness of the degrader at said other step. The energy attenuation can thus be varied by changing the angular position of the degrader.

A drawback of these known energy degraders is that they must have a large diameter in order to have steps of sufficiently small height ("H" in Fig.1c of WO-0038486) to obtain the resolution in energy variation which is required for particle therapy for instance.

As a consequence, these degraders have a large moment of inertia, so that it is difficult to rotate them quickly and/or with high accuracy with respect to the particle beam. Some recent applications require however to be able to change the energy of the particle beam very quickly, such as in a few tens of milliseconds for instance, and/or with high positional accuracy. This is for example the case with particle therapy systems, where a target, such as a tumour of a patient for example, is to be irradiated layer by layer with the particle beam, these layers being at different depths into the body of the patient. In such cases, it is desirable to be able to change the energy of the particle beam very quickly and/or very accurately when the system passes from one layer to another layer of the target.

Another drawback of their large diameter is that they require large quantities of expensive energy degrading material, which make them quite costly. A further drawback of their large diameter is that they are cumbersome and occupy lots of space, especially footprint space.

Another known rotating energy degrader is for example disclosed by R.E. Berg in "Rotating wedge cyclotron beam degrader" (Proceedings of the 7th International conference on cyclotrons and their applications; Zurich, Switzerland, 19-22 August 1975; pp.315-316).

It comprises a "comma"-shaped block of matter which is rotatable about an axis which is perpendicular to the "comma". The beam crosses the comma in a direction which is perpendicular to the rotation axis and hence enters into the "comma" at a convex side of the "comma" and exits out of the "comma" at a concave side of the "comma", or vice-versa. The energy attenuation is varied by changing the angular position of the "comma" with respect to the particle beam. When used for particle therapy applications, these energy degraders also require a large diameter and therefore present similar drawbacks as the one disclosed in WO-0038486, namely a high moment of inertia, high cost and large occupied volume.

Another drawback of this kind of energy degrader as that its angular length shall be smaller than 2*Pi radians, because otherwise the beam would pass through a large gap between two opposite arcs of the degrading material, which knowingly should be avoided.

### Summary of the invention

It is an object of the invention to address the drawbacks of the known energy degraders. It is a particular object of the invention to provide an energy degrader which is adapted to vary the energy of a particle beam more quickly and/or with higher accuracy than the known degraders.

A typical beam energy upstream (i.e. at an input) of an energy degrader according to the invention is in the MeV range, such as in the range of 150 MeV to 300 MeV for example, and a typical desired beam energy downstream (i.e. at an output) of an energy degrader according to the invention is also in the MeV range, such as in the range of 50 MeV to 230 MeV for an upstream energy of 230 MeV for example.

The invention is defined by the independent claims. The dependent claims define advantageous embodiments.

According to the invention, there is provided an energy degrader for attenuating the energy of a charged particle beam extracted from a particle accelerator, said energy degrader comprising a first energy attenuation member presenting a first beam entry face having the shape of part of a first helical surface having a first axis and a first beam exit face, and a second energy attenuation member, disjoint from the first energy attenuation member and presenting a second beam exit face having the shape of a part of a second helical surface having a second axis and a second beam entry face, the first axis being parallel to or coincident with the second axis.

The first beam exit face and the second beam entry face are facing each other, the first and second helical surfaces are continuous surfaces, and the first and second helical surfaces have the same handedness.

The energy degrader further comprises a drive assembly operably connected to the first and/or to the second energy attenuation members and configured for rotating the first and/or the second energy attenuation members about respectively the first axis and/or the second axis.

As will also appear hereafter from the figures showing exemplary embodiments of the invention, the degrader is hence spatially arranged so that the respective entry and exit faces of its two energy attention members are disposed in the following (continuous or discontinuous) sequence with respect to the path of a charged particle beam crossing it:
- the first beam entry face of the first energy attenuation member,
- the first beam exit face of the first energy attenuation member,
- the second beam entry face of the second energy attenuation member,
- the second beam exit face of the second energy attenuation member.

By "discontinuous sequence", it must be understood that additional attenuation material may be present in-between the beam exit and entry faces of respectively the first and second energy attenuation members, such as a flat material plate for example.

Thanks to the continuity of the first and second helical surfaces, the energy attenuation can be varied much more accurately and with a much higher resolution than with the known rotating degraders presenting discrete steps. This geometrical property, when combined with the first and second helical surfaces having the same handedness, also allows to use much steeper slopes for the entry and exit faces, yet keeping the statistical energy spread of the particles at the output of the degrader within acceptable limits, particularly for particle therapy applications. Indeed, the fact that these two helical surfaces have the same handedness will make that the various thicknesses of degrading material will be more homogeneous over the whole beam section than with conventional rotating degraders. And, by using steeper slopes, the degrader can be made more compact and with a smaller moment of inertia, thereby allowing to rotate it faster, so that energy attenuation can be varied faster. It also allows to use less attenuating material, which results in a cheaper degrader.

It is to be noted that a helical surface may have a close-up appearance of a helical staircase with very small steps, for example in case an energy attenuation member is made with a 3D printer, but that it is still to be considered as a continuous helical surface in case a minimum run (tread depth) of its steps is smaller than a minimum average beam diameter at a level where the beam crosses the helical surface (for example a minimum run of its steps which is smaller than 8 mm in case of an average beam diameter ranging between 8 mm and 30 mm when crossing the helical surface).

The drive assembly may be operably connected to the first energy attenuation member and configured for rotating it about the first axis, while the second energy attenuation member remains fixed.

However, the drive assembly is preferably operably connected to the first and to the second energy attenuation members and is configured for rotating both the first and the second energy attenuation members about respectively the first and the second axis. This allows to modify the angular position of both attenuation members and hence to better balance both attenuation members. More preferably, the drive assembly comprises a first motor operably connected to the first energy attenuation member and configured for rotating the first energy attenuation member about the first axis, and a second motor operably connected to the second energy attenuation member and configured for rotating the second energy attenuation member about the second axis.

With such a configuration, the first and second energy attenuation members can be moved independently from each other, which allows to better position the entry and exit faces of the energy attenuation members with respect to the particle beam along its path, more specifically with respect to a position of a waist in a particle beam profile and thus to reduce dispersion of the particle beam.

Preferably, the first and the second helical surfaces are cylindrical helical surfaces. More preferably, the first and the second helical surfaces have the same radius and the same pitch and the first rotation axis is coincident with the second rotation axis. This simplifies construction and operation of the energy degrader.

Alternatively, the first and the second helical surfaces are conical helical surfaces, the first and second energy attenuation members are right circular truncated cones whose truncated faces are the same and are facing each other, and the first axis (A1) of the first helical surface is coincident with the second axis (A1) of the second helical surface. Preferably, the truncated cones of the first and second energy attenuation members have the same aperture α, the first and second helical surfaces each have a slope which is equal to the aperture α, and the first and the second helical surfaces have the same pitch. This configuration allows to direct the particle beam with respect to the energy degrader in such a way that the particle beam enters the energy degrader perpendicularly to the first beam entry face and exits the energy degrader perpendicularly to the second beam exit face, which advantageously reduces an energy spread of the beam.

According to the invention, there is also provided a particle therapy system comprising a particle accelerator and an energy degrader as described herein, said energy degrader being positioned and oriented with respect to a particle beam extracted from the particle accelerator, in such a way that the extracted particle beam enters the energy degrader at the first beam entry face and exits the energy degrader at the second beam exit face.

In case the first and the second helical surfaces are conical helical surfaces, the energy degrader is preferably positioned and oriented with respect to the extracted particle beam in such a way that a normal vector to at least one of the first and second helical surfaces is parallel to the particle beam at the first beam entry face of the energy degrader.

Preferably, the particle accelerator is a fixed-energy accelerator, more preferably a cyclotron, even more preferably a synchrocyclotron.

### Short description of the drawings

These and further aspects of the invention will be explained in greater detail by way of example and with reference to the accompanying drawings in which:
- Fig.1: schematically shows a front view of an exemplary energy degrader according to the invention;
- Fig.2: schematically shows a top view of the energy degrader of Fig.1;
- Fig.3: schematically shows a partial sectional view of the energy degrader of Fig.1 at a high energy attention level;
- Fig.4: schematically shows a partial sectional view of the energy degrader of Fig.1 at a low energy attention level;
- Fig.5: schematically shows a front view of another exemplary energy degrader according to the invention;
- Fig.6: schematically shows a top view of the energy degrader of Fig.5;
- Fig.7: schematically shows a front view of another exemplary energy degrader according to the invention;
- Fig.8: schematically shows a top view of the energy degrader of Fig.7;
- Fig.9: schematically shows a perspective view of the energy degrader of Fig.7;
- Fig.10: schematically shows a partial sectional view of the energy degrader of Fig.7 at a high energy attention level;
- Fig.11: schematically shows a partial sectional view of the energy degrader of Fig.7 at a low energy attention level;
- Fig.12: schematically shows a particle therapy system comprising a particle accelerator and an energy degrader according to the invention;
- Fig.13: schematically shows another particle therapy system comprising a particle accelerator and an energy degrader according to the invention.

The drawings of the figures are neither drawn to scale nor proportioned. Generally, similar or identical components are denoted by the same reference numerals in the figures.

### Detailed description of embodiments of the invention

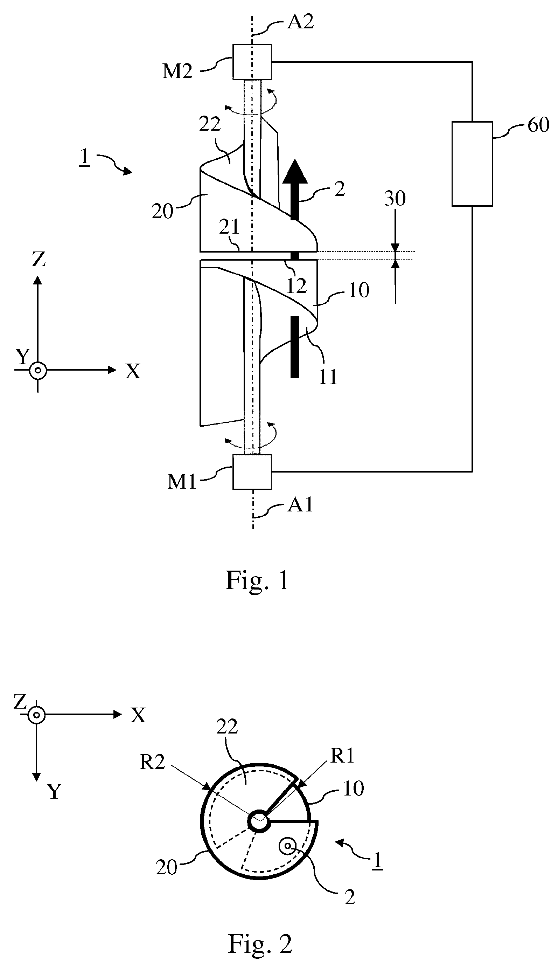

Fig.1 schematically shows a front view of an exemplary energy degrader (1) according to the invention, in an XYZ referential.

The energy degrader (1) comprises two disjoint energy attenuation members : a first energy attenuation member (10) and a second energy attenuation member (20).

The first energy attenuation member (10) presents a first beam entry face (11) having the shape of a part of a first continuous helical surface having a first axis (A1) and an opposed first beam exit face (12).

The second energy attenuation member (20) presents a second beam entry face (21), and an opposed second beam exit face (22) having the shape of part of a second continuous helical surface having a second axis (A2). The second axis (A2) is parallel to or coincident with the first axis (A1). The first and the second helical surfaces have the same handedness.

Preferably, the first and second helical surfaces each make a turn of less than or equal to 360° so that there is no overlap of their respective entry and exit faces in the axial direction. In other words, each of the first and second helical surfaces preferably have a height which is less than or equal to their respective pitch.

It is to be noted that a helical surface may have a close-up appearance of a helical staircase with very small steps, for example in case an energy attenuation member is made with a 3D printer, but that it is still to be considered as a continuous helical surface in case a minimum run (tread depth) of its steps is smaller than a minimum average beam diameter at a level where the beam crosses the helical surface (for example a minimum run of its steps which is smaller than 8 mm in case of an average beam diameter ranging between 8 mm and 30 mm when crossing the helical surface).

As can be seen on Fig.1, the first and second energy attenuation members (10, 20) are positioned with respect to each other in such a way that the first beam exit face (12) and the second beam entry face (21) are facing each other.

The energy degrader (1) further comprises a drive assembly which is operably connected to the first and/or the second energy attenuation members (10, 20). This drive assembly is configured for driving the first energy attenuation member (10) and/or the second energy attenuation member (20) into rotation about respectively the first axis (A1) and/or the second axis (A2).

The drive assembly may for example comprise a single motor (M1) as well as an optional transmission linking said single motor to the first energy attenuation member (10) so as to rotate the first energy attenuation member (10) about the first axis (A1), the second energy attenuation member (20) being fixed (not rotating).

Alternatively, the drive assembly may for example comprise a single motor as well as a transmission linking said single motor to respectively the first and the second energy attenuation members so as to rotate respectively the first and the second energy attenuation members, preferably in opposite directions (i.e. when the first energy attenuation member (10) is driven to rotate clockwise, the second energy attenuation member (20) will be driven to rotate anticlockwise and vice-versa).

Preferably, and as shown on Fig.1, the drive assembly comprises a first motor (M1) operably connected to the first energy attenuation member (10) for rotating the first energy attenuation member about the first axis (A1), and a second motor (M2) operably connected to the second energy attenuation member (20) for rotating the second energy attenuation member about the second axis (A2). The first and second motors may be stepper motors for example. Though not shown on Fig.1, the energy degrader (1) may further comprise (an) intermediary transmission(s) between the first and/or the second motors on the one hand and respectively the first and second energy attenuation members on the other hand, in order to adapt the speed and/or the torque applied by the motors to their corresponding energy attenuation members, and/or to improve the accuracy of the movements.

On Fig.1 is further shown a particle beam (2) when crossing the first and second energy attenuation members (10, 20). Given the geometry of these two attenuation members, it will be clear that an energy of an incoming particle beam will be more or less attenuated in function of the angular position(s) of the first and the second energy attenuation members with respect to the particle beam (2). A control unit (60), operably connected to the drive assembly (M1, M2), may be used to modify said angular positions, for example in function of energy attenuation settings received from a system using the energy degrader (1).

Fig.2 schematically shows a top view of the energy degrader (1) of Fig.1.

According to the invention, the first beam exit face (12) and the second beam entry face (21) may have various shapes, provided the first and second energy attenuation members (10, 20) are sufficiently spaced apart, so that one energy attenuation member is not hindered by the other energy attenuation member in the course of its rotation.

Preferably, the first beam exit face (12) has the shape of an annulus or a disk or a portion thereof, and the second beam entry face (21) has the shape of an annulus or a disk or a portion thereof. More preferably, the first beam exit face (12) is parallel to the second beam entry face (21). Even more preferably, the first beam exit face (12) and the second beam entry face (21) are perpendicular to the first and second rotation axes (A1, A2). With such a configuration, the gap (30) between the first beam exit face (12) and the second beam entry face (21) can be made small, which is desirable to reduce beam dispersion, particularly at high energy attenuation levels.

In such a case, the first and second energy attenuation members are preferably arranged in such a way that the gap (30) between the first beam exit face (12) and the second beam entry face (21) is smaller than 5 cm, preferably smaller than 1 cm, preferably smaller than 5 mm, preferably smaller than 1 mm. Preferably, the first beam exit face and the second beam entry face do not touch each other in order to avoid wear.

According to the invention, the first energy attenuation member (10) and/or the second energy attenuation member (20) are preferably made of beryllium or carbon graphite. More preferably, the first energy attenuation member (10) is made of the same material as the second energy attenuation member (20).

Preferably, the first and the second helical surfaces are cylindrical helical surfaces, as can be seen on the example of Figs. 1 and 2. Preferably, the first axis (A1) is the same as (coincident with) the second axis (A2). More preferably, the radius (R1) of the first helical surface is the same as the radius (R2) of the second helical surface. Even more preferably, the first and the second helical surfaces have the same pitch. Even more preferably, the first and second energy attenuation members (10, 20) are identical in shape and in size.

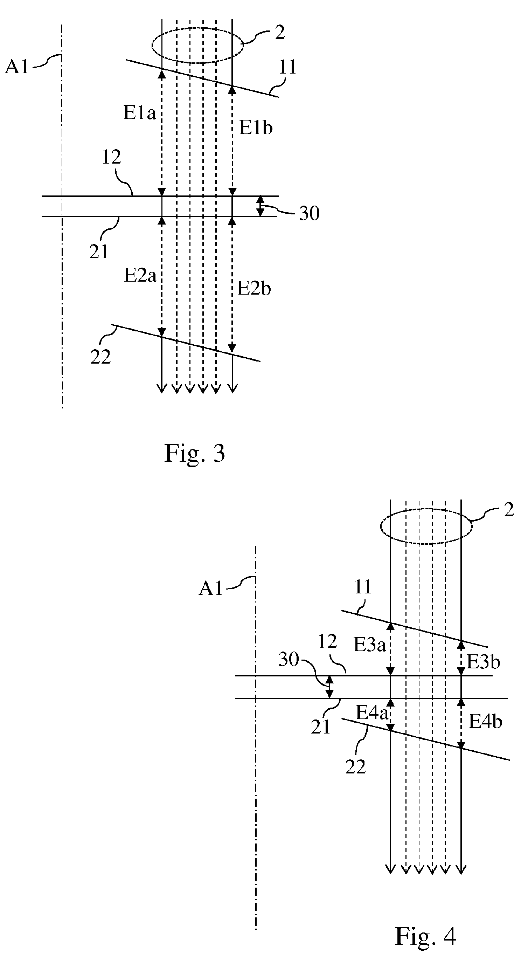

Fig.3 schematically shows a partial sectional and developed view of the energy degrader (1) of Fig.1 at a high energy attention level. In this exemplary representation, the first and second energy attenuation members (10, 20) have the same size and the same shape, which means that the first and second helical surfaces have the same radius, the same handedness and the same pitch.

The particle beam (2) is here shown enlarged in order to more clearly see its sectional size. As can be seen on Fig.3, a particle of the leftmost part of the beam (2) will travel through thicknesses E1 a and E2a of the energy attenuation members via a gap (30). A particle of the rightmost part of the beam (2) will travel through thicknesses E1 b and E2b of the energy attenuation members via the same gap (30). The gap (30) may for example be an air gap or a vacuum gap. A total attenuation of the energy of a particle may be estimated as the sum of the energy attenuations provided by the first and the second energy attenuation members along the path of the particle. In this exemplary configuration, we have that E1 a+E2a = E1 b+E2b, so that it will be understood that the energy of these two particles will be attenuated by the same amount. The same holds for the other particles of the beam (2). Such situation is desirable in order to limit the energy spread of the particles of the particle beam at the output of the degrader.

Fig.4 schematically shows a partial sectional and developed view of the energy degrader (1) of Fig.1 at a low energy attention level. This configuration may be obtained by rotating the first energy attenuation member (10) by a certain angle in the appropriate direction (in order to reduce the thicknesses E1 a and E1 b) and/or by rotating the second energy attenuation member (20) by a certain angle in the opposite direction (in order to reduce the thicknesses E2a and E2b). As can be seen on Fig.4, a particle of the leftmost part of the beam (2) will travel through thicknesses E3a and E4a of the energy attenuation members via the gap (30). A particle of the rightmost part of the beam (2) will travel through thicknesses E3b and E4b of the energy attenuation members via the same gap (30). It will therefore be understood that the energy of these two particles will be attenuated by the same amount. The same holds for the other particles of the beam (2). Such situation is desirable in order to limit the energy spread of the particles of the particle beam at the output of the degrader. Since E3a + E4a is smaller than E1 a + E2a, the energy of the beam will be less attenuated than with the arrangement of Fig.3.

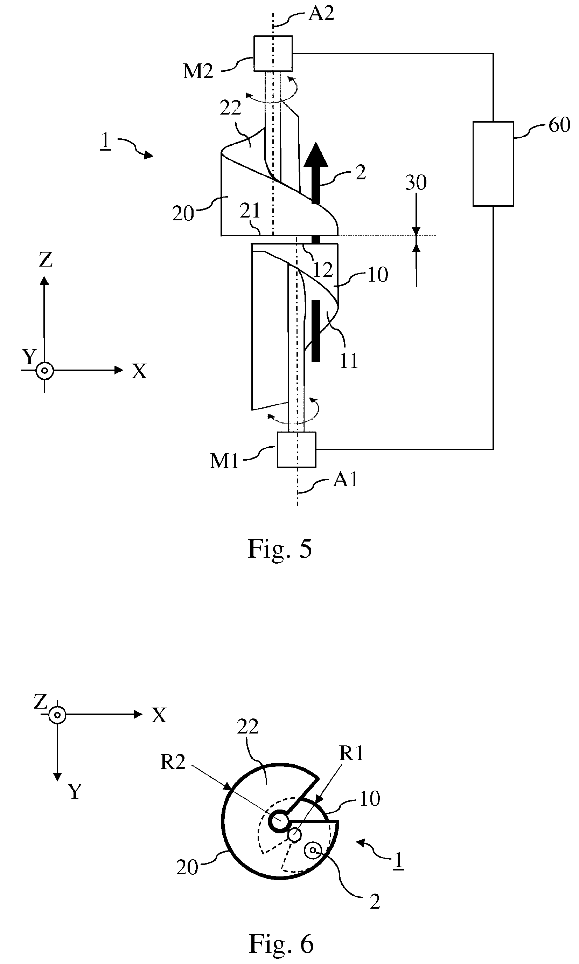

Fig. 5 schematically shows a front view of another exemplary energy degrader (1) according to the invention, in an XYZ referential. It is similar to the degrader of Fig.1, except that, in this case, the radius (R1) of the first helical surface is smaller than the radius (R2) of the second helical surface, and that the first axis (A1) is different from and parallel to the second axis (A2). Preferably, R1 < 0,5.R2, more preferably, R1 < 0,2.R2, even more preferably R1 < 0,1.R2. In this case, in order to have the same slope on both helical surfaces, the pitch of the first helical surface is preferably smaller than the pitch of the second helical surface.

Fig.6 schematically shows a top view of the energy degrader (1) of Fig.5, whereon one can also see that the first and second helical surfaces each preferably make a turn of less than or equal to 360° so that there is no overlap in the axial direction. In other words, each of the first and second helical surfaces preferably have a height which is less than or equal to their respective pitch.

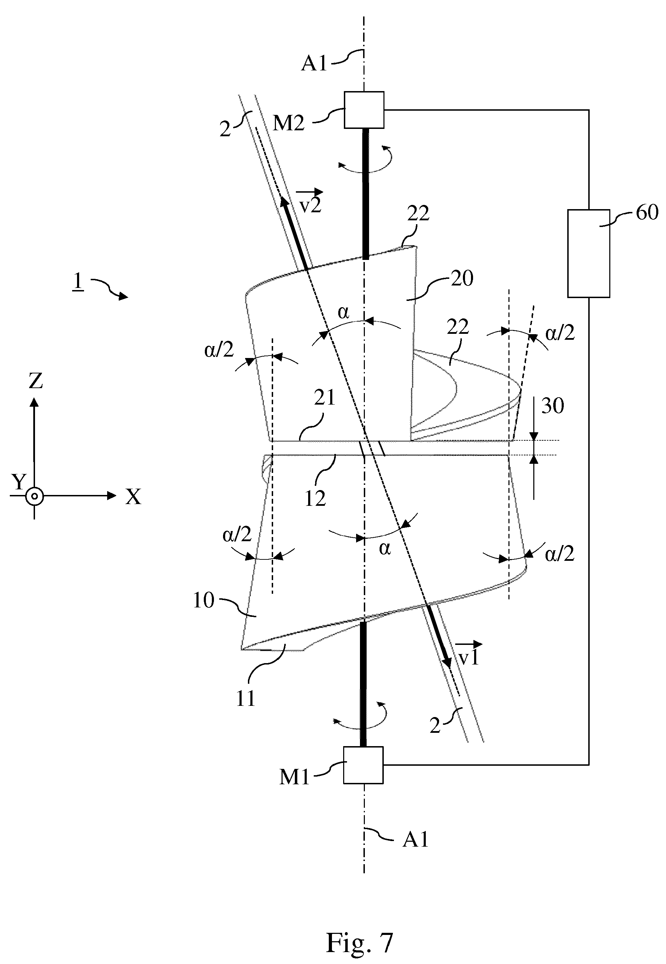

Fig.7 schematically shows a front view of another exemplary energy degrader (1) according to the invention in an XYZ referential. It is similar to the degrader of Fig.1, except that, in this case, the first and second helical surfaces are conical helical surfaces, and the first and second energy attenuation members are right circular truncated cones (10, 20) whose truncated faces (12, 21) are the same and are facing each other. The first axis (A1) of the first helical surface is coincident with the second axis (A1) of the second helical surface. Hence the two cones also have the same axis (A1). It is known in geometry that the aperture of a right circular cone is the maximum angle between two generatrix lines of the cone and that, if a generatrix makes an angle α/2 to the axis of the cone, the aperture of the cone is equal to α. Preferably, the first energy attenuation member (10) is designed in such a way that the aperture α of its truncated cone is equal to the slope of the first helical surface, so that a normal vector (v1) to the first helical surface makes the same angle α with the axis (A1) of the cone. In this case, the second energy attenuation member (20) is preferably designed in the same way, thus with the same angles α, as shown on Fig.7. The height of the truncated cone of the first energy attenuation member may be equal or different from the height of the truncated cone of the second energy attenuation member. Preferably, the first and second energy attenuation members (10, 20) are identical in shape and in size. Preferably, the first and second helical surfaces each make a turn of less than or equal to 360° so that there is no overlap in the axial direction. In other words, each of the first and second helical surfaces preferably have a height which is less than or equal to their respective pitch.

With such a geometry, as shown on Fig.7, a particle beam (2) whose path makes an angle α with the axis (A1) will enter into the degrader perpendicularly to the first beam entry face (11), will further pass through the matter of the two truncated cones (10, 20), and will thereafter exit the degrader perpendicularly to the second beam exit face (22), and this whatever the angular position of the first and second energy attenuation members. Having a particle beam entering and exiting the degrader perpendicularly to its entry and exit faces advantageously reduces dispersion of the beam.

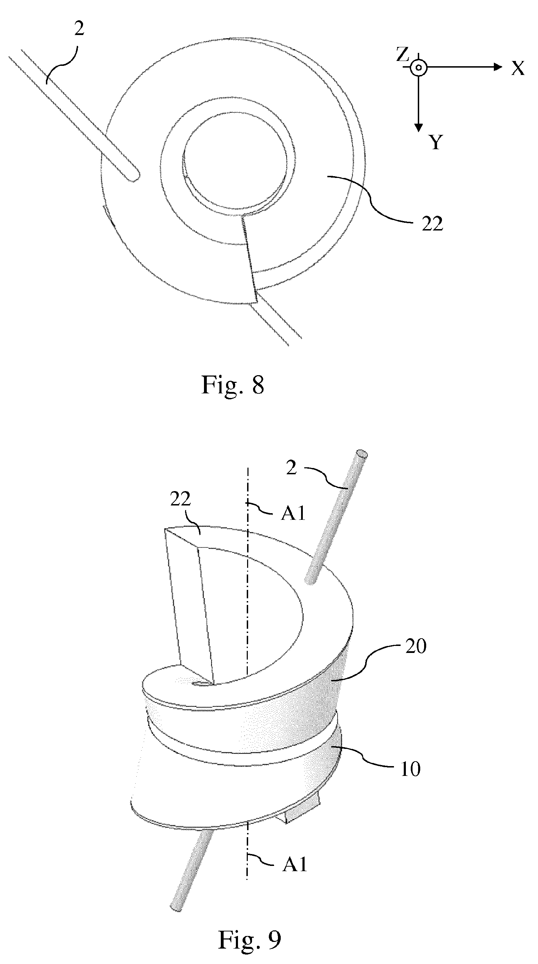

Fig.8 schematically shows a top view of the energy degrader of Fig. 7. The truncated cones (10, 20) may or may not be hollow. In order to reduce their moment of inertia, they are preferably hollow, as better seen shown on Fig.8 and Fig. 9.

Fig.9 schematically shows a perspective view of the energy degrader of Fig.7. For the sake of clarity, the drive assembly and its control unit are not shown on Figs. 8 and 9.

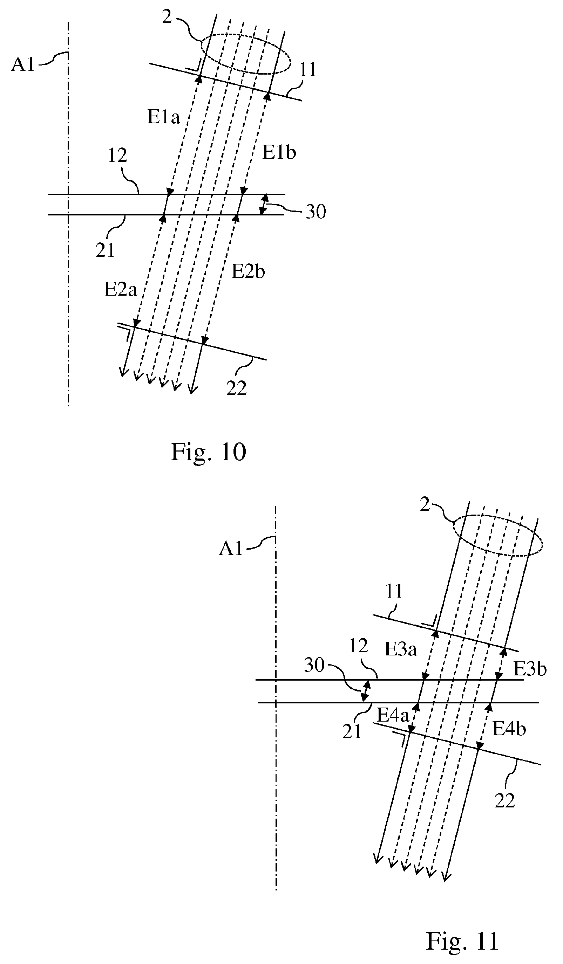

Fig.10 schematically shows a partial and developed sectional view of the energy degrader of Fig.7 at a high energy attention level, when crossed by particles of a particle beam (2) entering the first energy attenuation member (10) perpendicular to the first beam entry face (11). In this exemplary representation, the first and second energy attenuation members have the same size and the same shape. By analogy with the description of Fig.3, it will be understood that E1 a+E2a = E1 b+E2b, so that the energy of the corresponding two particles will be attenuated by the same amount. The same holds for the other particles of the beam (2).

Fig.11 schematically shows a partial and developed sectional view of the energy degrader of Fig.7 at a low energy attention level. This configuration may be obtained by rotating the first energy attenuation member (10) by a certain angle in the appropriate direction (in order to reduce the thicknesses E1 a and E1 b) and/or by rotating the second energy attenuation member (20) by a certain angle in the opposite direction (in order to reduce the thicknesses E2a and E2b). As for the case shown on Fig.10, the energy of the particles of the beam (2) will be attenuated by approximately the same amount.

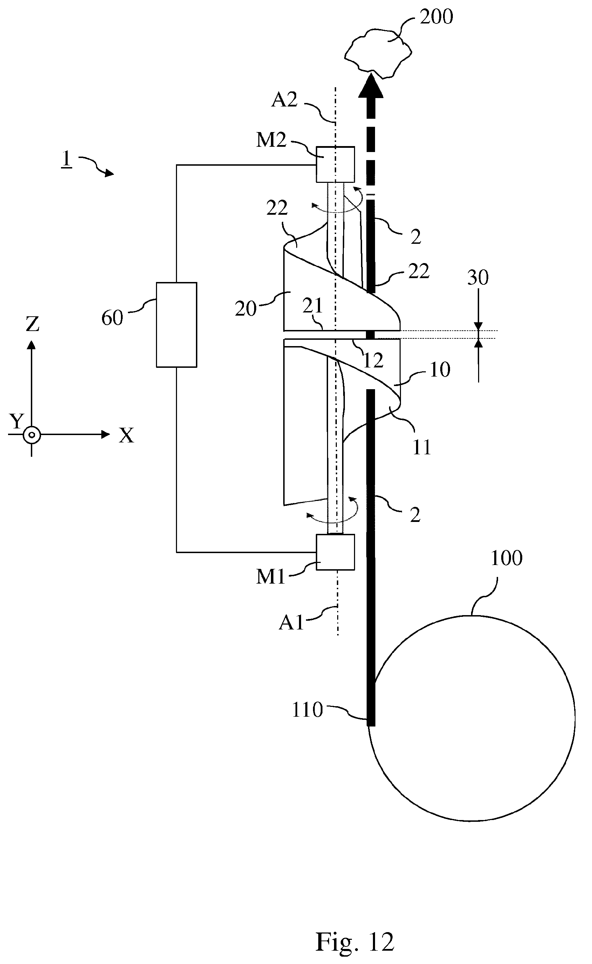

As schematically shown on Fig.12, the invention also concerns a particle therapy system configured for irradiating a target (200) with a charged particle beam (2). Said particle therapy system comprises a particle accelerator (100) configured for generating and extracting a beam (2) of charged particles, such as a beam of protons or carbon ions for example, and an energy degrader (1) as described hereinabove for attenuating the energy of said charged particle beam (2) before it reaches the target (200).

In this example, the energy degrader is positioned and oriented with respect to a path of the extracted particle beam (2) in such a way that the path of the extracted particle beam is parallel to the first axis (A1) at the first beam entry face (11) of the energy degrader.

For the sake of clarity, Fig. 12 does not necessarily show all components of a particle therapy system, which are well known from the prior art, but only those components which are necessary to understand the present invention.

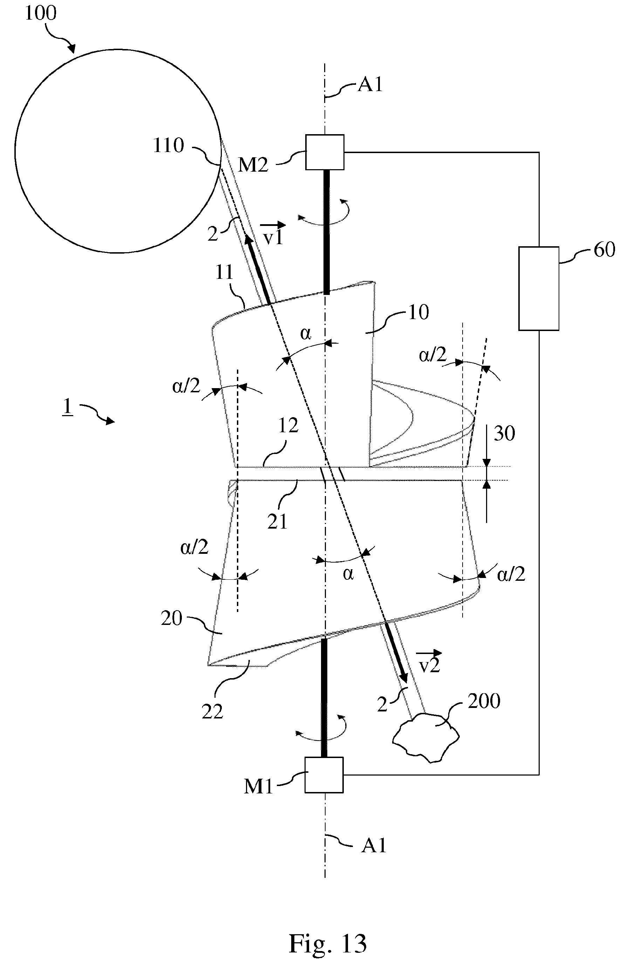

Fig.13 schematically shows another particle therapy system comprising a particle accelerator (100) and an energy degrader (1) according to the invention, and more specifically a degrader as shown in Fig.7. It is similar to the system of Fig. 12, except that in this case the energy degrader is positioned and oriented with respect to a path of the extracted particle beam (2) in such a way that the path of the extracted particle beam is parallel to a normal vector (v1) to the first helical surface at the first beam entry face (11) and/or parallel to a normal vector (v2) to the second helical surface (22) at the second beam exit face (22) of the energy degrader.

Preferably, the particle accelerator (100) is a fixed-energy accelerator, preferably a cyclotron, more preferably a synchrocyclotron.

Preferably, the particle accelerator (100) is configured for delivering at its output (110) a particle beam (2) whose maximal energy is comprised between 1 MeV and 500 MeV, preferably between 100 MeV and 300 MeV, more preferably between 200 MeV and 250 MeV.

In such a case, a typical desired beam energy at an output (22) of an energy degrader (1) according to the invention is also in the MeV range, such as in the range of 50 MeV to 230 MeV for an upstream energy of 230 MeV for example.

With these exemplary energies, which are common in particle therapy applications, one preferably has that:
- a minimal thickness (taken along the path of the particle beam) of the first energy attenuation member (10) lies in the interval [1 mm, 100 mm], more preferably [1 mm, 50 mm], even more preferably [1 mm, 10 mm]. The same holds for the second energy attenuation member (20),
- a maximal thickness (taken along the path of the particle beam) of the first energy attenuation member (10) lies in the interval [10 mm, 300 mm], more preferably [10 mm, 200 mm], even more preferably [10 mm, 100 mm]. The same holds for the second energy attenuation member (20), and
- a maximum diameter of the first energy attenuation member (10) lies in the interval [10 mm, 300 mm], more preferably [10 mm, 200 mm], even more preferably [10 mm, 150 mm]. The same holds for the second energy attenuation member (20).

The invention may also be described as follows: an energy degrader (1) for attenuating the energy of a charged particle beam (2) and comprising : a first energy attenuation member (10) presenting a beam entry face (11) having the shape of a part of a first helical surface, a second energy attenuation member (20) presenting a beam exit face (22) having the shape of a part of a second helical surface, said beam exit face being positioned downstream of said beam entry face with respect to the beam direction, and a drive assembly (M1, M2) for rotating the first and/or the second energy attenuation members about respectively a first and/or a second rotation axis (A1, A2). The first and second helical surfaces are continuous surfaces and have the same handedness, thereby allowing to build a more compact degrader with a smaller moment of inertia. More accurate and faster variation of the energy of the beam can hence be achieved.

The present invention has been described in terms of specific embodiments, which are illustrative of the invention and not to be construed as limiting. Reference numerals in the claims do not limit their protective scope.

Use of the verbs "to comprise", "to include", "to be composed of", or any other variant, as well as their respective conjugations, does not exclude the presence of elements other than those stated.

Use of the article "a", "an" or "the" preceding an element does not exclude the presence of a plurality of such elements.

## Claims

1. An energy degrader (1) for attenuating the energy of a charged particle beam extracted from a particle accelerator, said energy degrader comprising:
- a first energy attenuation member (10) presenting a first beam entry face (11) having the shape of part of a first helical surface having a first axis (A1) and a first beam exit face (12),
- a second energy attenuation member (20), disjoint from the first energy attenuation member (10) and presenting a second beam exit face (22) having the shape of a part of a second helical surface having a second axis (A2) and a second beam entry face (21),
the first axis (A1) being parallel to or coincident with the second axis (A2), the first beam exit face (12) and the second beam entry face (21) facing each other, the first and second helical surfaces being continuous surfaces, and the first and second helical surfaces have the same handedness, and
- a drive assembly operably connected to the first and/or to the second energy attenuation members (10, 20) and configured for rotating the first and/or the second energy attenuation members about respectively the first axis (A1) and/or the second axis (A2).

2. An energy degrader according to claim 1, **characterized in that** the drive assembly is operably connected to the first and to the second energy attenuation members (10, 20) and configured for rotating the first and the second energy attenuation members about respectively the first axis (A1) and the second axis (A2).

3. An energy degrader according to claim 2, **characterized in that** the drive assembly comprises:
- a first motor (M1) operably connected to the first energy attenuation member (10) and configured for rotating the first energy attenuation member about the first axis (A1), and
- a second motor (M2) operably connected to the second energy attenuation member (20) and configured for rotating the second energy attenuation member about the second axis (A2).

4. An energy degrader according to any of previous claims, **characterized in that** the first beam exit face (12) has the shape of an annulus or a disk or a portion thereof, **in that** the second beam entry face (21) has the shape of an annulus or a disk or a portion thereof, **in that** said first beam exit face (12) is parallel to said second beam entry face (21), and **in that** said first beam exit face and said second beam entry face are perpendicular to the first and second rotation axes (A1, A2).

5. An energy degrader according to claim 4, **characterized in that** a gap (30) between said first beam exit face (12) and said beam second entry face (21) is smaller than 10 mm, preferably smaller than 5 mm, more preferably smaller than 1 mm.

6. An energy degrader according to any of previous claims, **characterized in that** the first and second energy attenuation members (10, 20) are identical in shape and size.

7. An energy degrader according to any of claims 1 to 6, **characterized in that** the first and the second helical surfaces are cylindrical helical surfaces.

8. An energy degrader according to claim 7, **characterized in that** the first and the second helical surfaces have the same radius and the same pitch and **in that** the first rotation axis (A1) is coincident with the second rotation axis (A2).

9. An energy degrader according to claim 7, **characterized in that** the radius of the first helical surface is smaller than the radius of the second helical surface, **in that** the pitch of the first helical surface is smaller than the pitch of the second helical surface, and **in that** the first rotation axis (A1) is different from the second rotation axis (A2).

10. An energy degrader according to any of claims 1 to 6, **characterized in that** the first and the second helical surfaces are conical helical surfaces, **in that** the first and second energy attenuation members (10, 20) are right circular truncated cones whose truncated faces (12, 21) are the same and are facing each other, and **in that** the first axis (A1) of the first helical surface is coincident with the second axis (A1) of the second helical surface.

11. An energy degrader according to claim 10, **characterized in that** the truncated cones of the first and second energy attenuation members have the same aperture α, **in that** the first and second helical surfaces each have a slope which is equal to the said aperture α, and **in that** the first and the second helical surfaces have the same pitch.

12. A particle therapy system comprising a particle accelerator (100) and an energy degrader (1) according to any of previous claims, said energy degrader (1) being positioned and oriented with respect to a particle beam (2) extracted from the particle accelerator (100), in such a way that the extracted particle beam (2) enters the energy degrader (1) at the first beam entry face (11) and exits the energy degrader (1) at the second beam exit face (22).

13. A particle therapy system according to claim 12 comprising an energy degrader (1) according to any of claims 7 to 9, wherein the energy degrader is positioned and oriented with respect to a path of the extracted particle beam (2) in such a way that the path of the extracted particle beam (2) is parallel to the first axis (A1) at the first beam entry face (11) of the energy degrader.

14. A particle therapy system according to claim 12 comprising an energy degrader (1) according to any of claims 10 to 11, wherein the energy degrader is positioned and oriented with respect to a path of the extracted particle beam (2) in such a way that the path of the extracted particle beam is parallel to a normal vector (v1) to the first helical surface at the first beam entry face (11) of the energy degrader.

15. A particle therapy system according to any of claims 13 or 14, **characterized in that** the particle accelerator (100) is a fixed-energy accelerator, preferably a cyclotron, more preferably a synchrocyclotron.
